# EUROPEAN PATENT APPLICATION

(11) **EP 2 677 316 A1**
(43) Date of publication of application: **25.12.2013**
(21) Application number: 12747765.1
(22) Date of filing: 15.02.2012
(51) Int. Cl.: G01N 33/53, G01N 33/543, G01N 33/553

(54) **STREPTAVIDIN-BONDED MAGNETIC PARTICLES AND MANUFACTURING METHOD FOR SAME**

(30) Priority: 15.02.2011 JP 2011029330
(71) Applicant: Kyowa Medex Co., Ltd., Tokyo 104-6004 (JP)
(72) Inventor: ARAI, Nobuyuki, Shizuoka 411-0932 (JP); MATSUOKA, Yasuhiro, Shizuoka 411-0932 (JP); MORITA, Kazuki, Shizuoka 411-0932 (JP)
(74) Representative: Vossius & Partner
(86) International application number: PCT/JP2012/053463
(87) International publication number: WO 2012/111685

(57) **Abstract**

The present invention provides a streptavidin-coupled magnetic particle with high biotin-binding capacity, and a manufacturing method thereof. The streptavidin-coupled magnetic particle has a structure in which streptavidins are cross-linked with each other on a magnetic particle. A method for manufacturing the streptavidin-coupled magnetic particle includes the steps of:
(1) preparing a suspension containing magnetic particles having amino groups on their surface; and
(2) reacting the magnetic particles with streptavidin and glutaraldehyde by adding glutaraldehyde in the presence of streptavidin to the suspension prepared in step (1).

The streptavidin-coupled magnetic particle of the present invention, and the streptavidin-coupled magnetic particle manufactured by the manufacturing method of the present invention are useful in clinical diagnosis.

## Description

### Technical Field

The present invention relates to streptavidin-coupled magnetic particle and a manufacturing method thereof; a protein-coupled magnetic particle manufactured using the streptavidin-coupled magnetic particle, and a manufacturing method thereof; a method for measuring a component to be measured; and a reagent for measuring a component to be measured.

### Background Art

In diagnostic agents, magnetic particles are often used as solid phase carriers for detecting a substance to be measured such as hormones, cancer markers, and infection markers. In such measuring systems, antibodies, antigens, and the like (primary probes) are bound onto magnetic particles, and they are bound to substances to be measured in a specimen, and then the substances to be measured are further bound to secondary probes labeled with fluorescent substances, chemiluminescent substrates, enzymes, or such, and the substances to be measured are detected qualitatively or quantitatively.

Recently, there are needs to increase the sensitivity of examinations for early detection of diseases, for increasing the accuracy of examinations, for attending to highly sensitive markers in trace amounts, and such. There are also demands for promptness of examinations, due to a high-throughput processing accompanying establishment of testing centers, fast output of examination results aimed at providing services for patients, and such.

As a means to realize higher sensitivity and rapidity in measuring systems using such magnetic particles, the method of reacting a primary probe and a secondary probe in liquid phase and then binding this onto magnetic particles is often used. A representative example is a method in which a biotin-labeled primary probe, formed by binding a biotin to a primary probe, is reacted with a component to be measured in a sample and a secondary probe, to form a complex comprising the biotin-labeled primary probe, the component to be measured, and the secondary probe, and then an avidin-coupled magnetic particle is allowed to act on the complex to bind the complex onto a magnetic particle through avidin-biotin interaction.

Regarding such avidin-coupled magnetic particles, streptavidin-coupled magnetic particles using streptavidin, which has the same properties as avidin, are more useful. As with avidin, streptavidin binds very strongly to biotin, and has the property of being more resistant to denaturation than avidin. Furthermore, avidin has a basic isoelectric point whereas streptavidin has a weakly acidic or neutral isoelectric point; therefore, streptavidin is known to have the advantage of showing very low non-specific binding with other proteins. Streptavidin-coupled magnetic particles using this streptavidin are used for many purposes.

However, there is a limit to the amount of streptavidin that can be coupled onto the magnetic particles, and thus, a large amount of streptavidin-coupled magnetic particles has to be used to achieve the biotin-binding capacity needed for a reagent per test, which led to problems such as high manufacturing costs. Furthermore, the following problems have existed for measurements that use streptavidin-coupled magnetic particles:
(1) since magnetic particles precipitate under static conditions, they must be dispersed during use, and dispersing them requires time and effort when there is high particle content;
(2) when there is high particle content, the particles take up a large volume when brought to one side of the container using a magnet, and leads to decreased efficiency of washing out the reaction solution trapped inside them during B/F separation and washing; and
(3) turbidity caused by the color of the magnetic particles themselves increases when there is a large amount of magnetic particles during detection of the component to be measured, and for example, in detection by chemiluminescence and fluorescence, the sensitivity decreases due to optical shielding.

Under conditions where the amount of streptavidin-coupled magnetic particles is limited to give the minimum biotin-binding capacity required, there is a possibility that competition with biotin (vitamin H) present in a specimen inhibits the reaction for the measurement, and accurate test values may not be obtained. Biotin can be taken as a supplement or administered as a pharmaceutical agent, and such problems are often pointed out.

Meanwhile, several methods have been suggested as means to solve this problem. One of them is the method of decreasing the particle size to increase the surface area per weight of a magnetic particle. However, decreasing the particle size has problems. For example, the time taken to collect the particles using a magnet may be considerably lengthened and more particles may be carried away during the operation of dispensing-aspirating the washing solution in the washing step. Patent Document 1 describes, as a method for separating a substance to be detected in a specimen, a method of collecting magnetic particles from an aqueous solution by using magnetic particles modified on the surface with temperature-responsive polymers, wherein even magnetic particles having average particle sizes of 50 nm to 1,000 nm can be collected by particle aggregation of the temperature-responsive polymers. While such particles have advantages in the reaction due to the reduced size of the magnetic particles, they show non-specific adsorption due to the particle surface being covered by temperature-responsive polymers, and they require the step of replacing the conditions to special conditions for aggregation.

Furthermore, methods for making porous insoluble carriers to enlarge their surface area are also being suggested. For example, Patent Document 2 describes a method for chemically forming a porous layer at the outer layer of a magnetic particle. In this method, while binding capacity per surface area is increased in immunological reactions between antigens and antibodies, and in hybridization between DNAs or between DNA and RNA, efficiency of reactions in the pores is poor, and thus, it is difficult to achieve the expected performance.

### [Prior Art Documents]

### [Patent Documents]

[Patent Document 1] Japanese Patent Application *Kokai* Publication No. (JP-A) 2009-28711 (unexamined, published Japanese patent application)
[Patent Document 2] JP-A (Kokai) 2006-307126

### Summary of the Invention

### [Problems to be Solved by the Invention]

The objective of the present invention is to provide a streptavidin-coupled magnetic particle having high biotin-binding capacity, and a manufacturing method thereof. Furthermore, another objective of the present invention is to provide a protein-coupled magnetic particle manufactured using the streptavidin-coupled magnetic particle having high biotin-binding capacity and a manufacturing method thereof, a method for measuring a component to be measured, and a reagent for measuring a component to be measured.

### [Means for Solving the Problems]

The present inventors carried out dedicated examinations to solve the problems and discovered that streptavidin-coupled magnetic particles having high biotin-binding capacity can be obtained by reacting magnetic particles with streptavidin and glutaraldehyde through addition of glutaraldehyde, in the presence of streptavidin, to a suspension containing magnetic particles having amino groups on their surface; and the inventors completed the present invention. More specifically, the present invention relates to [1] to [12] below:
[1] a streptavidin-coupled magnetic particle, having a structure in which streptavidins are cross-linked with each other on a magnetic particle;
[2] the streptavidin-coupled magnetic particle of [1], which is manufactured by a method comprising the following steps of:
   (1) preparing a suspension comprising magnetic particles having amino groups on their surface; and
   (2) reacting the magnetic particles with streptavidin and glutaraldehyde by adding glutaraldehyde in the presence of streptavidin to the suspension prepared in step (1);
[3] the streptavidin-coupled magnetic particle of [2], which is manufactured by a method further comprising the following step (3):
   (3) reacting the streptavidin-coupled magnetic particles prepared in step (2) with a reducing agent;
[4] a protein-coupled magnetic particle, which is manufactured using the streptavidin-coupled magnetic particle of any one of [1] to [3] and a biotinylated protein;
[5] a method for measuring a component to be measured in a sample, which uses the protein-coupled magnetic particle of [4];
[6] a method for measuring a component to be measured in a sample, which uses the streptavidin-coupled magnetic particle of any one of [1] to [3] and a biotinylated protein;
[7] a reagent for measuring a component to be measured in a sample, which comprises the protein-coupled magnetic particle of [4];
[8] a reagent for measuring a component to be measured in a sample, which comprises the streptavidin-coupled magnetic particle of any one of [1] to [3] and a biotinylated protein;
[9] a method for manufacturing streptavidin-coupled magnetic particles, which comprises the following steps of:
   (1) preparing a suspension comprising magnetic particles having amino groups on their surface; and
   (2) reacting the magnetic particles with streptavidin and glutaraldehyde by adding glutaraldehyde in the presence of streptavidin to the suspension prepared in step (1);
[10] the manufacturing method of [9], further comprising the following step (3):
   (3) reacting the streptavidin-coupled magnetic particles prepared in step (2) with a reducing agent;
[11] a method for manufacturing protein-coupled magnetic particles, which comprises the following steps of:
   (1) preparing a suspension comprising magnetic particles having amino groups on their surface;
   (2) preparing streptavidin-coupled magnetic particles by adding glutaraldehyde in the presence of streptavidin to the suspension prepared in step (1); and
   (3) reacting the streptavidin-coupled particles prepared in step (2) with a biotinylated protein; and
[12] a method for manufacturing protein-coupled magnetic particles, which comprises the following steps of:
   (1) preparing a suspension comprising magnetic particles having amino groups on their surface;
   (2) preparing streptavidin-coupled magnetic particles by adding glutaraldehyde in the presence of streptavidin to the suspension prepared in step (1);
   (3) reacting the streptavidin-coupled magnetic particles prepared in step (2) with a reducing agent; and
   (4) reacting the streptavidin-coupled magnetic particles prepared in step (3) with a biotinylated protein.

### [Effects of the Invention]

The present invention provides a streptavidin-coupled magnetic particle having high biotin-binding capacity and a manufacturing method thereof, a protein-coupled magnetic particle manufactured using the streptavidin-coupled magnetic particle and a manufacturing method thereof, a method for measuring a component to be measured, and a reagent for measuring a component to be measured. The streptavidin-coupled magnetic particle and protein-coupled magnetic particle manufactured by the manufacturing method of the present invention, as well as the method for measuring a component to be measured and the reagent for measuring a component to be measured of the present invention, are useful in clinical diagnosis.

### Brief Description of the Drawings

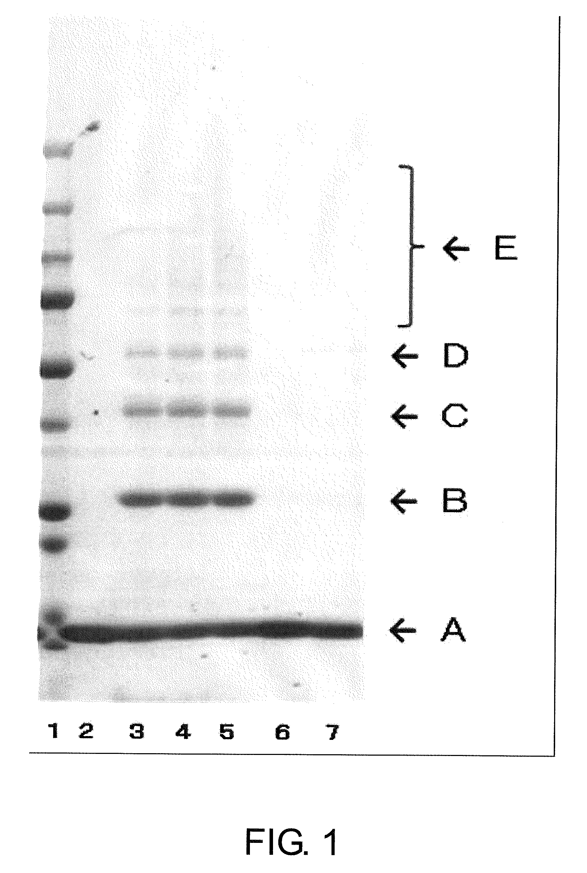

Fig. 1 shows an SDS-PAGE electrophoretic profile showing the structures of streptavidins on the magnetic particles of streptavidin-coupled magnetic particles of the present invention and of commercially available streptavidin-coupled magnetic particles. The lanes show the following: lane 1, molecular markers; lane 2, streptavidin; lane 3, streptavidin-coupled magnetic particles having a biotin-binding capacity of 2.61 pmol/mm²; lane 4, streptavidin-coupled magnetic particles having a biotin-binding capacity of 4.95 pmol/mm²; lane 5, streptavidin-coupled magnetic particles having a biotin-binding capacity of 6.76 pmol/mm²; lane 6, commercially available streptavidin-coupled magnetic particle Dynabeads T1 (manufactured by Dynal); and lane 7, commercially available streptavidin-coupled magnetic particle BE-M08/10 (manufactured by Merck). Band A represents monomers, band B represents dimers, band C represents trimers, band D represents tetramers, and band E represents cross-linked structures of higher order.

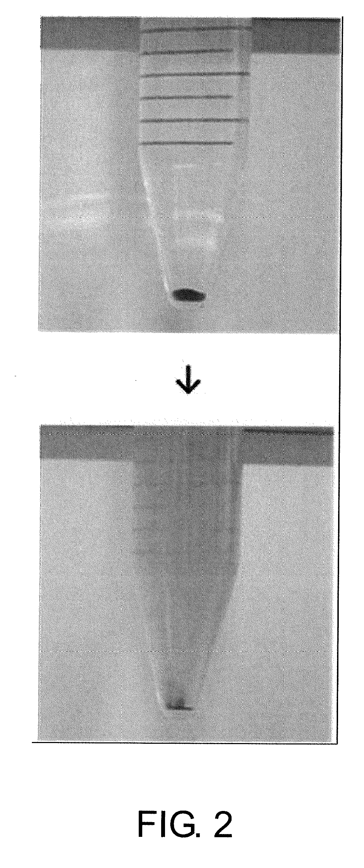

Fig. 2 shows photographs depicting the dispersibility of the streptavidin-coupled magnetic particles of the present invention. The top photograph shows the static state of the streptavidin-coupled magnetic particles, and the bottom photograph shows the dispersed state of the streptavidin-coupled magnetic particles after mixing by inversion for 25 times.

### Mode for Carrying Out the Invention

### 1. Streptavidin-coupled magnetic particles

Streptavidin-coupled magnetic particles of the present invention have a structure in which streptavidins are cross-linked with each other on magnetic particles. Streptavidins form a tetrameric structure and the monomers are bound to each other through non-covalent bonds. In the streptavidin-coupled magnetic particles of the present invention, these streptavidins having tetrameric structure are covalently bonded with each other via glutaraldehyde to form a cross-linked structure on the magnetic particles. Streptavidins are bound to the amino group of the magnetic particles via glutaraldehyde. More specifically, a portion of the streptavidins having tetrameric structure is bound to the amino group of the magnetic particles via glutaraldehyde. The cross-linked structure of streptavidin can be confirmed by SDS-PAGE (sodium dodecyl sulfate - polyacrylamide gel electrophoresis), gel filtration HPLC, and such, for example, by placing the streptavidin-coupled magnetic particles in 1% SDS solution and subjecting them to treatment at 60°C for one hour to dissociate the binding between subunits of streptavidins coupled to the magnetic particles. SDS-PAGE is a method for separating proteins depending on their size by electrophoresis, and is a method for separating and identifying proteins from the obtained electrophoretic profile by denaturing a sample using SDS and then subjecting the denatured protein to polyacrylamide gel electrophoresis. SDS-PAGE is not particularly limited as long as it is a method that can confirm the cross-linked structures of streptavidin, and an example is the method described in "Baio Jikken Irasutoreiteddo (Bio-experiment Illustrated) 5" (Cell Engineering Supplement, Shujunsha).

In SDS-PAGE, streptavidins having tetrameric structure lose their tetrameric structure by denaturation treatment in the presence of SDS. If the streptavidins on the magnetic particles are not in the form of cross-linked structures, the degradation product that can be obtained by denaturation treatment in the presence of SDS will be solely the streptavidin-derived monomers. On the other hand, if the streptavidins on the magnetic particles are in the form of cross-linked structures, denaturation treatment in the presence of SDS should yield, in addition to the streptavidin-derived monomers, dimers, trimers, and higher-order multimers that had participated in the cross-linked structures of streptavidins. Therefore, in case bands resulting from streptavidin-derived monomers, dimers, trimers, and higher-order multimers are observed by SDS-PAGE, this means that cross-linked structures of streptavidins are formed on the magnetic particles.

The streptavidin-coupled magnetic particles of the present invention have high biotin-binding capacity because they have a structure in which streptavidins are cross-linked with each other on the magnetic particles. The biotin-binding capacity per particle of the streptavidin-coupled magnetic particles of the present invention is ordinarily 0.5-10 pmol/mm², preferably 2-9 pmol/mm², and particularly preferably 4-8 pmol/mm². The streptavidin-coupled magnetic particles of the present invention also have good dispersibility, which is an excellent property. The dispersibility of streptavidin-coupled magnetic particles can be evaluated, for example, by storing the particles in a cuvette and after mixing the precipitated streptavidin-coupled magnetic particles by inversion confirming the condition inside the cuvette through visual observation.

The biotin-binding capacity per particle of the streptavidin-coupled magnetic particles of the present invention can be measured by any method as long as it is a method that can measure biotin-binding capacity. For example, the binding capacity can be calculated by reacting a given amount of fluorescence-labeled biotin with a given amount of streptavidin-coupled magnetic particles, collecting the streptavidin-coupled magnetic particles using a magnet, then collecting a given amount of a supernatant, measuring the fluorescence intensity of the collected supernatant, and correlating the obtained measured values with a calibration curve prepared in advance which indicates the relationship between fluorescence intensity and biotin concentration.

In the streptavidin-coupled magnetic particles of the present invention, the streptavidin can be naturally derived or genetically engineered, and genetically engineered streptavidin is preferred.

In the streptavidin-coupled magnetic particles of the present invention, the magnetic particles to which streptavidin is fixed are those having amino groups on their surface. The magnetic particles having amino groups on their surface in the present invention are not particularly limited as long as they can produce the streptavidin-coupled magnetic particles of the present invention. Examples of the particle structure of the magnetic particles having amino groups on their surface in the present invention include magnetic particles with core-shell structure wherein the inner part of the particles contain a magnetic substance and the outer layer is composed of organic polymer and such; magnetic particles having a structure which does not include an outer layer and has magnetic substances dispersed heterogeneously in organic polymer; and magnetic particles in a cluster state composed only of magnetic substances. Specific examples of the magnetic particles having amino groups on their surface (commercially available products) include amino group-type Estapor magnetic particles (manufactured by Merck).

Magnetic substances included in the magnetic particles are preferably superparamagnetic microparticles with little residual magnetization, and for example, various ferrites such as triiron tetraoxide (Fe₃O₄) and γ-diiron trioxide (γ-Fe₂O₃), metals such as iron, manganese, cobalt, and chromium, alloys of such metals, and such are used.

The content of magnetic substances in magnetic particles composed of organic polymers and magnetic substances is preferably 10 wt% or more, and more preferably 30-60 wt% of the total weight of the magnetic particles.

Examples of the shape of the magnetic particles include spherical and needle shape, and spherical shapes are preferred. The particle size of the magnetic particles is for example 0.1-5 µm, and is preferably 0.5-3 µm.

### 2. Methods for manufacturing streptavidin-coupled magnetic particles

The method for manufacturing streptavidin-coupled magnetic particles of the present invention is a manufacturing method comprising the following steps of:
(1) preparing a suspension comprising magnetic particles having amino groups on their surface; and
(2) reacting the magnetic particles with streptavidin and glutaraldehyde by adding glutaraldehyde, in the presence of streptavidin, to the suspension prepared in step (1).

The streptavidin-coupled magnetic particles of the present invention can be manufactured by using the manufacturing methods of the present invention.

### (1) Step of preparing a suspension containing magnetic particles

The suspension which contains magnetic particles having amino groups on their surface can be prepared by adding magnetic particles having amino groups on their surface to an aqueous medium, or by adding to an aqueous medium magnetic particles having amino groups on their surface. Examples of the magnetic particles having amino groups on their surface include the aforementioned magnetic particles having amino groups on their surface. The aqueous medium is not particularly limited as long as it is an aqueous medium that can suspend magnetic particles having amino groups on their surface, and examples include distilled water, purified water, and buffer. The pH of the aqueous medium is normally pH4.5 to 7, and is preferably pH5 to 6. When using a buffer solution as the aqueous medium, it is desirable to use a buffer appropriate for the pH which is to be set. Examples of the buffer used for the buffer solution include an acetate buffer, a citrate buffer, a succinate buffer, a phosphate buffer, and a Good's buffer.

Examples of the Good's buffer include 2-morpholinoethanesulfonic acid (MES), bis(2-hydroxyethyl)iminotris(hydroxymethyl)methane (Bis-Tris), piperazine-*N,N*'-bis(2-ethanesulfonic acid) (PIPES), *N*-(2-acetamido)-2-aminoethanesulfonic acid (ACES), 3-morpholino-2-hydroxypropanesulfonic acid (MOPSO), *N,N-*bis(2-hydroxyethyl)-2-aminoethanesulfonic acid (BES), 3-morpholinopropanesulfonic acid (MOPS), *N*-[tris(hydroxymethyl)methyl]-2-aminoethanesulfonic acid (TES), 2-[4-(2-hydroxyethyl)-1-piperazinyl)ethanesulfonic acid (HEPES), 3-[*N*,*N-*bis(2-hydroxyethyl)amino]-2-hydroxypropanesulfonic acid (DIPSO), and *N-*[tris(hydroxymethyl)methyl]-2-hydroxy-3-aminopropanesulfonic acid (TAPSO).

The magnetic particles having amino groups on their surface to be suspended in an aqueous medium may be pre-washed. The magnetic particles having amino groups on their surface can be washed, for example, by adding the magnetic particles having amino groups on their surface to a dispersion liquid in a container, dispersing the magnetic particles having amino groups on their surface, then collecting the magnetic particles having amino groups on their surface using a magnet, and removing the dispersion liquid remaining in the container by aspiration. Examples of the dispersion liquid include aqueous solutions containing a surfactant. The pH of the dispersion liquid is ordinarily pH4.5 to 7, and preferably pH5 to 6. Examples of the aqueous medium used for the aqueous solution include the aforementioned aqueous media. The surfactant is not particularly limited as long as it enables dispersion of the magnetic particles, and examples include anionic surfactants, cationic surfactants, amphoteric surfactants, and nonionic surfactants. The concentration of the surfactant in the dispersion solution is not particularly limited as long as it is a concentration that can disperse the magnetic particles, and it is, for example, 0.01% to 5.0%.

### (2) Step of reacting the magnetic particles with glutaraldehyde and streptavidin

Step (2) is a step for forming a structure in which streptavidins are cross-linked on magnetic particles by reacting the magnetic particles with streptavidin and glutaraldehyde through addition of glutaraldehyde, in the presence of streptavidin, to the suspension containing the magnetic particles having amino groups on their surface prepared in step (1). The structure of cross-linked streptavidins is formed by covalent bonding between streptavidins via glutaraldehyde. Herein, addition of glutaraldehyde in the presence of streptavidin means that streptavidin is present when glutaraldehyde is added, and includes cases where streptavidin is added to the suspension of magnetic particles and then glutaraldehyde is sequentially added to it, and cases in which glutaraldehyde and streptavidin are added simultaneously to the suspension of magnetic particles.

The amount of glutaraldehyde added is not particularly limited as long as it is an amount that can produce the streptavidin-coupled magnetic particles of the present invention, and is ordinarily 0.1 mg to 1.0 mg, and preferably 0.35 mg to 0.6 mg with respect to 100 mg of magnetic particles.

The amount of streptavidin added is not particularly limited as long as it is an amount that can produce the streptavidin-coupled magnetic particles of the present invention, and is ordinarily 0.2 mg to 25 mg, and preferably 10 mg to 15 mg with respect to 100 mg of magnetic particles.

The concentration of magnetic particles in the reaction solution is not particularly limited as long as it is a concentration that can produce the streptavidin-coupled magnetic particles of the present invention, and is ordinarily 20 mg/mL to 80 mg/mL, and preferably 40 mg/mL to 60 mg/mL.

The reaction temperature is ordinarily 0°C to 40°C, preferably 27.5°C to 37.5°C, and particularly preferably 35°C. The reaction time is ordinarily 4 to 24 hours, preferably 8 to 20 hours, and particularly preferably 18 hours.

While the reaction mixture obtained in step (2) itself can be used as the streptavidin-coupled magnetic particles of the present invention, magnetic particles obtained by collecting the magnetic particles in the reaction mixture obtained in step (2) using a magnet, removing the solution but not the magnetic particles, and then washing the particles using a washing solution can also be used as the streptavidin-coupled magnetic particles of the present invention. The washing solution is not particularly limited, as long as it is a washing solution that can wash substances other than the streptavidin-coupled magnetic particles of the present invention, and examples include the aforementioned aqueous media. In addition, aqueous media containing proteins and/or antiseptics can be used as the washing solution. The proteins include, for example, bovine serum albumin (BSA). The antiseptics include, for example, sodium azide. Furthermore, the washed magnetic particles can be stored after suspending them in a storage solution. The storage solution is not particularly limited as long as it is a solution that enables stable storage of the streptavidin-coupled magnetic particles of the present invention, and examples include aqueous solutions of neutral to weakly acidic buffer containing proteins such as bovine serum albumin (BSA).

Methods for manufacturing the streptavidin-coupled magnetic particles of the present invention can further include a reduction step after step (2). The reduction step is a step of reacting the streptavidin-coupled magnetic particles prepared in step (2) with a reducing agent. According to step (2), streptavidins with cross-linked structures are formed on the magnetic particles and since the cross-linked streptavidins contain Schiff bases (imines), reducing the Schiff bases (imines) using a reducing agent allows the cross-linked structures to become more stable.

The reaction mixture of step (2) itself or the washed magnetic particles can be used as the streptavidin-coupled magnetic particles in the reduction step. The solvent used in the reaction between streptavidin-coupled magnetic particles and a reducing agent is not particularly limited as long as it is a solvent that can allow the reduction reaction to proceed, and examples include the aforementioned dispersion liquids. Furthermore, a dispersion liquid containing an organic solvent can also be used as a solvent in the reduction reaction. The organic solvent is not particularly limited as long as it is an organic solvent that is soluble in water and can allow the reduction reaction to proceed, and examples include methanol, ethanol, and tetrahydrofuran. The reducing agent is not particularly limited as long as it is a reducing agent that can reduce Schiffbases (imines) and maintain the cross-linked structure, and examples include borane-based reducing agents. Examples of the borane-based reducing agent include 2-picoline borane and sodium borohydride. The amount of reducing agent added is ordinarily 0.0001 to 0.1 (w/w)%, preferably 0.0005 to 0.05 (w/w)%, and particularly preferably 0.001 (w/w)% of the magnetic particles.

The reaction temperature for the reduction reaction is ordinarily 30°C to 50°C, preferably 35°C to 45°C, and particularly preferably 40°C. The reaction time for the reduction reaction is ordinarily two to ten days, preferably five to eight days, and particularly preferably six days.

After the reduction reaction, the magnetic particles can be separated from solution components other than the magnetic particles using a magnet. For example, the separated magnetic particles can be washed using the aforementioned dispersion liquid or a diluted storage solution, and they can be stored as a suspension in a storage solution. The storage solution is not particularly limited as long as it is a solution that enables stable storage of the streptavidin-coupled magnetic particles of the present invention, and examples include the aforementioned storage solution.

### 3. Methods for manufacturing protein-coupled magnetic particles

The protein-coupled magnetic particles of the present invention are manufactured using streptavidin-coupled magnetic particles of the present invention and a biotinylated protein. Proteins are bound onto the magnetic particles through interaction between streptavidin on the magnetic particles and biotin bound to the protein.

For example, the protein-coupled magnetic particles of the present invention can be manufactured by a method comprising the steps described below. Specific embodiments of the method for manufacturing protein-coupled magnetic particles of the present invention are shown below.
- Method 1 for manufacturing protein-coupled magnetic particles
   (1) preparing a suspension comprising magnetic particles having amino groups on their surface;
   (2) preparing streptavidin-coupled magnetic particles by adding glutaraldehyde, in the presence of streptavidin, to the suspension prepared in step (1); and
   (3) reacting the streptavidin-coupled particles prepared in step (2) with a biotinylated protein.
- Method 2 for manufacturing protein-coupled magnetic particles
   (1) preparing a suspension comprising magnetic particles having amino groups on their surface;
   (2) preparing streptavidin-coupled magnetic particles by adding glutaraldehyde, in the presence of streptavidin, to the suspension prepared in step (1);
   (3) reacting the streptavidin-coupled magnetic particles prepared in step (2) with a reducing agent; and
   (4) reacting the streptavidin-coupled magnetic particles prepared in step (3) with a biotinylated protein.

The reaction between streptavidin-coupled magnetic particles and biotinylated proteins can be performed under any condition as long as it is a condition, under which the protein is bound onto the magnetic particles. The reaction temperature is ordinarily 25°C to 50°C, and preferably 30°C to 40°C. The reaction time is ordinarily 30 minutes to 24 hours, and preferably 2 to 18 hours.

Examples of the protein include antibodies that bind to the component to be measured, and competitive substances that compete with the component to be measured in the antigen-antibody reaction. Examples of the competitive substance include a component to be measured, and a substance containing an epitope recognized by an antibody that binds to the component to be measured. Specific examples of the protein include IgG, anti-IgG antibody, IgM, anti-IgM antibody, IgA, anti-IgA antibody, IgE, anti-IgE antibody, apoprotein AI, anti-apoprotein AI antibody, apoprotein AII, anti-apoprotein AII antibody, apoprotein B, anti-apoprotein B antibody, apoprotein E, anti-apoprotein E antibody, rheumatoid factor, anti-rheumatoid factor antibody, D-dimer, anti-D-dimer antibody, oxidized LDL, anti-oxidized LDL antibody, glycated LDL, anti-glycated LDL antibody, glycoalbumin, anti-glycoalbumin antibody, triiodothyronine (T3), anti-T3 antibody, total thyroxine (T4), anti-T4 antibody, pharmaceutical agents (for example, antiepileptic drugs), antibodies that bind to pharmaceutical agents, C-reactive protein (CRP), anti-CRP antibody, cytokines, antibodies that bind to cytokines, α-fetoprotein (AFP), anti-AFP antibody, carcinoembryonic antigen (CEA), anti-CEA antibody, CA19-9, anti-CA19-9 antibody, CA15-3, anti-CA15-3 antibody, CA-125, anti-CA-125 antibody, PIVKA-II, anti-PIVKA-II antibody, parathyroid hormone (PTH), anti-PTH antibody, human chorionic gonadotrophin (hCG), anti-hCG antibody, thyroid-stimulating hormone (TSH), anti-TSH antibody, insulin, anti-insulin antibody, C-peptide, anti-C-peptide antibody, estrogen, anti-estrogen antibody, fibroblast growth factor-23 (FGF-23), anti-FGF-23 antibody, glutamate decarboxylase (GAD), anti-GAD antibody, pepsinogen, anti-pepsinogen antibody, hepatitis B virus (HBV) antigen, anti-HBV antibody, hepatitis C virus (HCV) antigen, anti-HCV antibody, human T-cell leukemia virus type 1 (HTLV-I) antigen, anti-HTLV-I antibody, human immunodeficiency virus (HIV) antigen, anti-HIV antibody, influenza virus antigen, anti-influenza virus antibody, Mycobacterium tuberculosis antigen (TBGL), anti-Mycobacterium tuberculosis antibody, mycoplasma antigen, anti-mycoplasma antibody, hemoglobin A1c, anti-hemoglobin A1c antibody, atrial natriuretic peptide (ANP), anti-ANP antibody, brain natriuretic peptide (BNP), anti-BNP antibody, troponin T, anti-troponin T antibody, troponin I, anti-troponin I antibody, creatinine kinase-MB (CK-MB), anti-CK-MB antibody, myoglobin, anti-myoglobin antibody, L-FABP, anti-L-FABP antibody, H-FABP, anti-H-FABP antibody, CCP antigen, anti-CCP antibody, SP-D, anti-SP-D antibody, antibodies that bind to mycotoxins [such as deoxynivalenol (DON), nivalenol (NIV), and T-2 toxin (T2)], antibodies that bind to endocrine disruptors [such as bisphenol A, nonylphenol, dibutyl phthalate, polychlorinated biphenyls (PCBs), dioxins, *p,p*'-dichlorodiphenyltrichloroethane, and tributyltin], antibodies that bind to steroid hormones (such as aldosterone and testosterone), fungi such as *Escherichia coli,* antibodies that bind to fungi-derived proteins, food allergens, allergens such as Acari, and anti-allergen antibodies.

In addition to biotinylated proteins, biotinylated hydrocarbon compounds and biotinylated nucleic acids can be used. Hydrocarbon compound-coupled magnetic particles can be produced by reacting the streptavidin-coupled magnetic particles of the present invention with a biotinylated hydrocarbon compound. Furthermore, nucleic acid-coupled magnetic particles can be produced by reacting the streptavidin-coupled magnetic particles of the present invention with a biotinylated nucleic acid.

Examples of the hydrocarbon compound in the biotinylated hydrocarbon compounds include mycotoxins [such as deoxynivalenol (DON), nivalenol (NIV), and T-2 toxin (T2)], endocrine disruptors [such as bisphenol A, nonylphenol, dibutyl phthalate, polychlorinated biphenyls (PCBs), dioxins, *p,p*'-dichlorodiphenyltrichloroethane, and tributyltin], and steroid hormones (such as aldosterone and testosterone).

Examples of the nucleic acid in the biotinylated nucleic acids include DNA, RNA, aptamer, and derivatives, thereof.

### 4. Methods for measuring a component to be measured

An embodiment of the method for measuring a component to be measured of the present invention is a method for measuring a component to be measured in a sample using the streptavidin-coupled magnetic particles of the present invention and a biotinylated protein. Another embodiment of the method for measuring a component to be measured of the present invention is a method for measuring a component to be measured in a sample using the protein-coupled magnetic particles of the present invention. For the measurement method of the present invention, any method may be used as long as it is a method used in ordinary immunoassays, and examples include the sandwich method and the competition method.

Examples of the protein in biotinylated proteins and protein-coupled magnetic particles include antibodies that bind to a component to be measured and competitive substances that compete with a component to be measured in an antigen-antibody reaction. Examples of the competitive substance include components to be measured and substances containing an epitope recognized by an antibody that binds to the component to be measured. Specific examples of the protein include the aforementioned proteins.

The sample in the present invention is not particularly limited as long as it is a sample that enables measurement of a component to be measured of the present invention. Examples include whole blood, plasma, serum, cerebrospinal fluid, saliva, amniotic fluid, urine, sweat, and pancreatic juice, and plasma and serum are preferred.

The component to be measured is not particularly limited as long as it can be measured by the measurement method of the present invention, and examples include IgG, IgM, IgA, IgE, apoprotein AI, apoprotein AII, apoprotein B, apoprotein E, rheumatoid factor, D-dimer, oxidized LDL, glycated LDL, glycoalbumin, triiodothyronine (T3), total thyroxine (T4), pharmaceutical agents (for example, antiepileptic drugs), C-reactive protein (CRP), cytokines, α-fetoprotein (AFP), carcinoembryonic antigen (CEA), CA19-9, CA15-3, CA-125, PIVKA-II, parathyroid hormone (PTH), human chorionic gonadotrophin (hCG), thyroid-stimulating hormone (TSH), insulin, C-peptide, estrogen, fibroblast growth factor-23 (FGF-23), anti-glutamate decarboxylase (GAD) antibody, pepsinogen, hepatitis B virus (HBV) antigen, anti-HBV antibody, hepatitis C virus (HCV) antigen, anti-HCV antibody, human T-cell leukemia virus type 1 (HTLV-I) antigen, anti-HTLV-I antibody, anti-human immunodeficiency virus (HIV) antibody, influenza virus antigen, anti-influenza virus antibody, anti-Mycobacterium tuberculosis antibody, Mycobacterium tuberculosis antigen (TBGL), anti-mycoplasma antibody, hemoglobin A1c, atrial natriuretic peptide (ANP), brain natriuretic peptide (BNP), troponin T, troponin I, creatinine kinase-MB (CK-MB), myoglobin, L-FABP, H-FABP, anti-CCP antibody, SP-D, mycotoxins [such as deoxynivalenol (DON), nivalenol (NIV), and T-2 toxin (T2)], endocrine disruptors [such as bisphenol A, nonylphenol, dibutyl phthalate, polychlorinated biphenyls (PCBs), dioxins, *p,p*'-dichlorodiphenyltrichloroethane, and tributyltin], steroid hormones (such as aldosterone and testosterone), fungi such as *Escherichia coli,* food allergens, allergens such as Acari, and anti-allergen antibodies.

### (I) Methods for measuring a component to be measured using the streptavidin-coupled magnetic particles of the present invention and a biotinylated protein

### (I-1) Sandwich method

Examples of the method for measuring a component to be measured in a sample by the Sandwich method include methods comprising the following steps of:
(1) forming, on magnetic particles, immunocomplexes comprising a first antibody that binds to the component to be measured, the component to be measured, and a second antibody that binds to the component to be measured by reacting, in an aqueous medium, a component to be measured in a sample with the streptavidin-coupled magnetic particles of the present invention, a biotinylated first antibody in which biotin is bound to a first antibody that binds to the component to be measured, and a second antibody that binds to the component to be measured;
(2) collecting the magnetic particles in the reaction mixture after step (1) using magnetic force, and separating the magnetic particles collected by magnetic force from the other components; and
(3) measuring the immunocomplexes on the magnetic particles separated in step (2).

Instead of the first and second antibodies, a first antibody fragment and a second antibody fragment can be used. Examples of the fragment of an antibody include Fab, F(ab')₂, and Fab'.

The aqueous medium is not particularly limited as long as it is an aqueous medium that enables the antigen-antibody reaction, and examples include the aforementioned aqueous media.

In step (1), the component to be measured in a sample reacts in an aqueous medium with streptavidin-coupled magnetic particles, a biotinylated first antibody, and a second antibody to form on the magnetic particles an immunocomplex comprising the first antibody, the component to be measured, and the second antibody.

Herein, the reaction of the component to be measured in a sample with streptavidin-coupled magnetic particles, a biotinylated first antibody, and a second antibody may be any reaction as long as it is a reaction that forms on the magnetic particles an immunocomplex comprising the first antibody, the component to be measured, and the second antibody. For example, the component to be measured in a sample can be reacted with streptavidin-coupled magnetic particles and a biotinylated first antibody to form on the magnetic particles an immunocomplex of the first antibody and the component to be measured, then this can be reacted with a second antibody; or alternatively, the component to be measured in a sample can be reacted simultaneously with streptavidin-coupled magnetic particles, a biotinylated first antibody, and a second antibody. In case formation of an immunocomplex of the first antibody and the component to be measured on the magnetic particles is followed by reaction of the immunocomplex with the second antibody, a washing step can be set up after the formation of the immunocomplex. The washing of magnetic particles after formation of an immunocomplex of the first antibody and the component to be measured is not particularly limited as long as it is a washing that can retain the immunocomplex on the magnetic particles. Examples include methods of washing the magnetic particles by removing components other than the magnetic particles from the reaction mixture after the formation of the immunocomplex of the first antibody and the component to be measured on the magnetic particles, and adding a washing solution to the reaction vessel containing the remaining magnetic particles; and methods of washing the magnetic particles by adding a washing solution to the reaction mixture after the reaction and, at the same time, removing the components other than the magnetic particles. Removal of components other than the magnetic particles can be carried out, for example, by collecting the magnetic particles by magnetic force, and then aspirating the remaining components. Examples of the washing solution include the aforementioned aqueous media, and aqueous media produced by adding a surfactant to the aforementioned aqueous media. Examples of the surfactant include non-ionic surfactants such as Tween 20.

The reaction temperature in the antigen-antibody reaction of step (1) is not particularly limited as long as it is a temperature that enables measurement of the component to be measured of the present invention, and it is ordinarily, 0°C to 50°C, preferably 4°C to 45°C, and particularly preferably 20°C to 40°C. The reaction time is not particularly limited as long as it is a time that enables measurement of the component to be measured of the present invention, and it is ordinarily, five minutes to one hour, and preferably five minutes to 20 minutes.

In step (2), the magnetic particles after step (1), or specifically, the magnetic particles to which immunocomplexes comprising the first antibody, the component to be measured, and the second antibody are bound, are collected by magnetic force, and the collected magnetic particles are separated from the other components. The magnetic force for collecting the magnetic particles is not particularly limited as long as it is a magnetic force that enables the measurement of the component to be measured of the present invention. The separation of the magnetic particles collected by magnetic force from the other components is not particularly limited as long as it is a separation that enables the measurement of the component to be measured of the present invention.

After step (2) or simultaneously with step (2), a step of washing the magnetic particles to which immunocomplexes comprising the first antibody, the component to be measured, and the second antibody are bound can be included. The magnetic particles can be washed, for example, by the aforementioned methods.

In step (3), the component to be measured in a sample can be measured by measuring the immunocomplexes on the magnetic particles separated by step (2). Examples of the method for measuring the immunocomplexes include the following methods.

### (1) In case the second antibody is not labeled

The immunocomplexes on the separated magnetic particles can be measured by reacting a labeled third antibody, in which a label is bound to a third antibody that binds to the second antibody, with the magnetic particles to which the immunocomplexes comprising the first antibody, the component to be measured, and the second antibody are bound to form, on the magnetic particles, immunocomplexes comprising the first antibody, the component to be measured, the second antibody, and the third antibody, and then measuring the label in the immunocomplexes. A third antibody fragment can be used instead of the third antibody. Examples of the third antibody that binds to the second antibody include antibodies or a fragment thereof that bind to the Fc region of the second antibody. Measurement of the label is not particularly limited as long as it is a method that enables measurement of an immunocomplex on the separated magnetic particles. Examples include measurement of chemiluminescence, measurement of fluorescence, and measurement of absorbance. Examples of the antibody fragment include Fab, F(ab')₂, and Fab'.

### (2) In case the second antibody that binds to the component to be measured is labeled

The immunocomplexes on the separated magnetic particles can be measured by measuring the label in the immunocomplexes formed on the magnetic particles and comprising the first antibody, the component to be measured, and the labeled second antibody. Measurement of the label is not particularly limited as long as it is a method that enables measurement of an immunocomplex on the separated magnetic particles. Examples include measurement of chemiluminescence, measurement of fluorescence, and measurement of absorbance.

### (A) Measurement of chemiluminescence

Measurement of chemiluminescence can be carried out by methods such as the following.

### (A-1) In case the label is an enzyme

In case the label is an enzyme, for example, the measurement can be carried out by allowing a substrate that produces light upon reacting with the enzyme to act on a labeled antibody or a fragment thereof, and measuring the intensity of the produced light (hυ) using a luminescence intensity meter or such. The enzyme is not particularly limited as long as it can react with a substrate of the enzyme and produce light, and examples include alkaline phosphatase, peroxidase, β-D-galactosidase, and luciferase.

In case of using alkaline phosphatase as the enzyme, examples of the substrate of alkaline phosphatase which reacts with alkaline phosphatase to produce light include
3-(2'-spiroadamantane)-4-methoxy-4-(3'-phosphoryloxy)phenyl-1,2-dioxetane disodium salt (AMPPD),
2-chloro-5- {4-methoxyspiro[1,2-dioxetane-3,2'-(5'-chloro)tricyclo[3.3.1.13,7]cane]-4-yl}phenyl phosphate disodium salt (CDP-Star™),
3-{4-methoxyspiro[1,2-dioxetane-3,2'-(5'-chloro)tricyclo[3.3.1.13,7]decane]-4-yl}phenylphosph ate disodium salt (CSPD™), [10-methyl-9(10H)-acridinylidene]phenoxymethylphosphate disodium salt (Lumigen™ APS-5), and
9-(4-chlorophenylthiophosphoryloxymethylidene)-10-methylacridan disodium salt.

In case of using peroxidase as the enzyme, examples of the substrate of peroxidase which reacts with peroxidase to produce light include combinations of hydrogen peroxide with a luminescent compound (for example, a luminol compound or a lucigenin compound).

In case of using β-D-galactosidase as the enzyme, examples of the substrate of β-D-galactosidase which reacts with β-D-galactosidase to produce light include Galacton-Plus (manufactured by Applied Biosystems).

In case of using luciferase as the enzyme, examples of the substrate of luciferase which reacts with luciferase to produce light include luciferin and coelenterazine.

### (A-2) In case the label is a luminescent substance

In case the label is a luminescent substance, for example, the measurement can be carried out by measuring the intensity of light originated from the luminescent substance in the formed immunocomplexes using a luminescence intensity meter or the like. The luminescent substance is not particularly limited as long as it is a luminescent substance that enables the measurement of the present invention, and examples include acridinium and derivatives thereof, ruthenium complex compounds, and lophine.

### (B) Measurement of fluorescence

Fluorescence can be measured by methods such as the following.

### (B-1) In case the label is an enzyme

In case the label is an enzyme, for example, the measurement can be carried out by allowing a substrate that produces fluorescence upon reacting with the enzyme to act on a labeled antibody or a fragment thereof, and measuring the intensity of the produced fluorescence using a fluorescence intensity meter or such. The enzyme is not particularly limited as long as it can react with a substrate of the enzyme to produce fluorescence, and examples include peroxidase, β-D-galactosidase, and β-glucuronidase.

In case of using peroxidase as the enzyme, examples of the substrate of peroxidase which reacts with peroxidase to produce fluorescence include combinations of hydrogen peroxide and a fluorescent compound (for example, 4-hydroxyphenylacetic acid, 3-(4-hydroxyphenyl)propionic acid, or coumarin).

In case of using β-D-galactosidase as the enzyme, examples of the substrate of β-D-galactosidase which reacts with β-D-galactosidase to produce fluorescence include 4-methylumbeliferyl-β-D-galactopyranoside or an analog thereof.

In case of using β-glucuronidase as the enzyme, examples of the substrate of β-glucuronidase which reacts with β-glucuronidase to produce fluorescence include TokyoGreen™-βGluU (manufactured by Sekisui Medical Co. Ltd.).

### (B-2) In case the label is a fluorescent substance

In case the label is a fluorescent substance, for example, the measurement can be carried out by measuring the intensity of fluorescence originated from the fluorescent substance in the formed immunocomplexes using a fluorescence intensity meter or the like. The fluorescent substance is not particularly limited as long as it is a fluorescent substance that enables the measurement of the present invention, and examples include fluorescein isothiocyanate (FITC), rhodamine B-isothiocyanate (RITC), quantum dot (Science, 281, 2016-2018, 1998), phycobiliproteins such as phycoerythrin, green fluorescent protein (GFP), red fluorescent protein (RFP), yellow fluorescent protein (YFP), and blue fluorescent protein (BFP).

### (C) Measurement of absorbance

Absorbance can be measured by methods such as the following.

### (C-1) In case the label is an enzyme

In case the label is an enzyme, for example, the measurement can be carried out by allowing a substrate that forms a dye upon reacting with the enzyme to act on a labeled antibody or a fragment thereof, and measuring the absorbance of the formed dye using a spectrophotometer, a multi-well plate reader, or the like. The enzyme is not particularly limited as long as it can react with the substrate of the enzyme to form a dye, and examples include peroxidase.

In case peroxidase is used as the enzyme, examples of the substrate of peroxidase which reacts with peroxidase to form a dye include combinations of hydrogen peroxide and an oxidative coloring type chromogen. Examples of the oxidative coloring type chromogen include leuco-type chromogens and oxidative coupling-coloring chromogens.

The leuco-type chromogen is a substance that is converted into a dye by itself in the presence of hydrogen peroxide and a peroxidative substance such as peroxidase. Specific examples include tetramethylbenzidine, *o*-phenylenediamine, 10-*N*-carboxymethylcarbamoyl-3,7-bis(dimethylamino)-10H-phenothiazine (CCAP), 10-*N-*methylcarbamoyl-3,7-bis(dimethylamino)-10H-phenothiazine (MCDP), 10-*N-*(carboxymethylaminocarbonyl)-3,7-bis(dimethylamino)-10H-phenothiazine sodium salt (DA-67), *N*,*N*,*N*',*N*',*N*",*N*"-hexa(3-sulfopropyl)-4,4',4",-triamino-triphenylmethane (TPM-PS), *N*-(carboxymethylaminocarbonyl)-4,4'-bis(dimethylamino)diphenylamine sodium salt (DA-64), 4,4'-bis(dimethylamino)diphenylamine, and bis[3-bis(4-chlorophenyl)methyl-4-dimethylaminophenyl]amine (BCMA).

The oxidative coupling-coloring chromogen is a substance that forms a dye by oxidative coupling of two compounds in the presence of hydrogen peroxide and a peroxidative substance such as peroxidase. Examples of the combination of two compounds include a combination of couplers and anilines (Trinder reagent), and a combination of couplers and phenols. Examples of the coupler include 4-aminoantipyrine (4-AA) and 3-methyl-2-benzothiazolinonehydrazine. Examples of the anilines include *N*-(3-sulfopropyl)aniline, *N*-ethyl-*N*-(2-hydroxy-3-sulfopropyl)-3-methylaniline (TOOS), *N*-ethyl-*N*-(2-hydroxy-3-sulfopropyl)-3,5-dimethylaniline (MAOS), *N*-ethyl-*N*-(2-hydroxy-3-sulfopropyl)-3,5-dimethoxyaniline (DAOS), *N*-ethyl-*N*-(3-sulfopropyl)-3-methylaniline (TOPS), *N*-(2-hydroxy-3-sulfopropyl)-3,5-dimethoxyaniline (HDAOS), *N,N-*dimethyl-3-methylaniline, *N,N-*bis(3-sulfopropyl)-3,5-dimethoxyaniline, *N*-ethyl-*N*-(3-sulfopropyl)-3-methoxyaniline, *N*-ethyl-*N*-(3-sulfopropyl)aniline, *N*-ethyl-*N*-(3-sulfopropyl)-3,5-dimethoxyaniline, *N-*(3-sulfopropyl)-3,5-dimethoxyaniline, *N*-ethyl-*N*-(3-sulfopropyl)-3,5-dimethylaniline, *N*-ethyl-*N*-(2-hydroxy-3-sulfopropyl)-3-methoxyaniline, *N*-ethyl-*N*-(2-hydroxy-3-sulfopropyl)aniline, *N-*ethyl-*N-*(3-methylphenyl)-*N'*-succinylethylenediamine (EMSE), *N*-ethyl-*N*-(3-methylphenyl)-*N*'-acetylethylenediamine, and *N*-ethyl-*N*-(2-hydroxy-3-sulfopropyl)-4-fluoro-3,5-dimethoxyaniline (F-DAOS). Examples of the phenols include phenol, 4-chlorophenol, 3-methylphenol, and 3-hydroxy-2,4,6-triiodobenzoic acid (HTIB).

The concentration of the component to be measured in a sample, based on the measured values obtained from measurements of the immunocomplexes formed on the magnetic particles, can be determined by methods such as the following.

The concentration of the component to be measured in a sample to be measured is determined by performing the above-mentioned steps (1) to (3) using the component to be measured with a known concentration, producing a calibration curve showing the relationship between the concentration of the component to be measured and the measured value (the amount of information derived from the label), and then taking measurements using the sample to be measured, and correlating the measured values with the produced calibration curve.

### (I-2) Competition method 1

Examples of the method for measuring the component to be measured in a sample by a competition method include methods comprising the following steps:
(1) reacting, in an aqueous medium, the component to be measured in a sample with streptavidin-coupled magnetic particles of the present invention, a biotinylated antibody in which biotin is bound to an antibody that binds to both the component to be measured and the competitive substance, and a labeled competitive substance in which a label is bound to a competitive substance; and
(2) measuring the label in the immunocomplex of the labeled competitive substance and the antibody that binds to the component to be measured, which was formed in step (1).

After step (1), a step of washing the magnetic particles that have been applied for the antigen-antibody reaction can be set up. The magnetic particles can be washed, for example, by the aforementioned washing methods.

Measurement of the label in step (2) can be carried out, for example, by the aforementioned methods.

The concentration of the component to be measured in a sample, based on the measured values obtained from measurements of the immunocomplexes formed on the magnetic particles, can be determined by methods such as the following.

The concentration of the component to be measured in a sample to be measured is determined by performing the above-mentioned steps (1) and (2) using the component to be measured with a known concentration, producing a calibration curve showing the relationship between the concentration of the component to be measured and the measured value (the amount of information derived from the label), and then taking measurements using the sample to be measured, and correlating the measured values with the produced calibration curve.

### (I-3) Competition method 2

Examples of the method for measuring the component to be measured in a sample by a competition method include methods comprising the following steps:
(1) reacting, in an aqueous medium, the component to be measured in a sample with streptavidin-coupled magnetic particles of the present invention, a biotinylated competitive substance in which biotin is bound to the competitive substance, and a labeled antibody in which a label is bound to an antibody that binds to both the component to be measured and the competitive substance; and
(2) measuring the label in the immunocomplex of the competitive substance and the labeled antibody, which was formed in step (1).

After step (1), a step of washing the magnetic particles that have been applied for the antigen-antibody reaction can be set up. The magnetic particles can be washed, for example, by the aforementioned washing methods.

Measurement of the label in step (2) can be carried out, for example, by the aforementioned methods.

The concentration of the component to be measured in a sample, based on the measured values obtained from measurements of the immunocomplexes formed on the magnetic particles, can be determined by methods such as the following.

The concentration of the component to be measured in a sample to be measured is determined by performing the above-mentioned steps (1) and (2) using the component to be measured with a known concentration, producing a calibration curve showing the relationship between the concentration of the component to be measured and the measured value (the amount of information derived from the label), and then taking measurements using the sample to be measured, and correlating the measured values with the produced calibration curve.

### (II) Methods for measuring a component to be measured using the protein-coupled magnetic particles of the present invention

### (II-1) Sandwich method

Examples of the method for measuring a component to be measured in a sample by the Sandwich method include methods that use an antibody that binds to the component to be measured as the protein in the protein-coupled magnetic particles of the present invention, and comprise the following steps of:
(1) forming, on magnetic particles, immunocomplexes comprising a first antibody that binds to the component to be measured, the component to be measured, and a second antibody that binds to the component to be measured by reacting, in an aqueous medium, a component to be measured in a sample with magnetic particles to which a first antibody that binds to the component to be measured is fixed, and a second antibody that binds to the component to be measured;
(2) collecting the magnetic particles in the reaction mixture after step (1) using magnetic force, and separating the magnetic particles collected by magnetic force from the other components; and
(3) measuring the immunocomplexes on the magnetic particles separated in step (2).

Instead of the first and second antibodies, a first antibody fragment and a second antibody fragment can be used. The aqueous medium is not particularly limited as long as it is an aqueous medium that enables the antigen-antibody reaction, and examples include the aforementioned aqueous media.

In step (1), the component to be measured in a sample reacts in an aqueous medium with the first antibody on the magnetic particles and the second antibody to form on the magnetic particles an immunocomplex comprising the first antibody, the component to be measured, and the second antibody.

Herein, the reaction of the component to be measured in a sample with a first antibody on the magnetic particles and a second antibody may be any reaction as long as it is a reaction that forms on the magnetic particles an immunocomplex comprising the first antibody, the component to be measured, and the second antibody. For example, the component to be measured in a sample can be reacted with the first antibody on the magnetic particles to form on the magnetic particles an immunocomplex of the first antibody and the component to be measured, then this can be reacted with the second antibody; or alternatively, the component to be measured in a sample can be reacted simultaneously with the first antibody on the magnetic particles and the second antibody. In case formation of an immunocomplex of the first antibody and the component to be measured on the magnetic particles is followed by reaction of the immunocomplex with the second antibody, a washing step can be set up after the formation of the immunocomplex. The washing of magnetic particles after formation of the immunocomplex of the first antibody and the component to be measured is not particularly limited as long as it is a washing that can retain the immunocomplex on the magnetic particles, and examples include the above-mentioned washing methods.

The reaction temperature in the antigen-antibody reaction of step (1) is not particularly limited as long as it is a temperature that enables measurement of the component to be measured of the present invention, and it is ordinarily, 0°C to 50°C, preferably 4°C to 45°C, and particularly preferably 20°C to 40°C. The reaction time is not particularly limited as long as it is a time that enables measurement of the component to be measured of the present invention, and it is ordinarily, five minutes to one hour, and preferably five to 20 minutes.

Step (2) and step (3) are the same as the aforementioned step (2) and step (3) of (I-1).

The concentration of the component to be measured in a sample, based on the measured values obtained from measurements of the immunocomplexes formed on the magnetic particles, can be determined, for example, by methods similar to the aforementioned case of (I-1).

### (II-2) Competition method 1

Examples of the method for measuring a component to be measured in a sample by a competition method include methods that use an antibody that binds to both the component to be measured and a competitive substance as the protein in the protein-coupled magnetic particles of the present invention, and comprising the following steps of:
(1) reacting, in an aqueous medium, the component to be measured in a sample with magnetic particles to which an antibody that binds to both the component to be measured and the competitive substance is fixed, and a labeled competitive substance in which a label is bound to a competitive substance; and
(2) measuring the label in the immunocomplex of the labeled competitive substance and the antibody that binds to the component to be measured, which was formed in step (1).

After step (1), a step of washing the magnetic particles that have been applied for the antigen-antibody reaction can be set up. The magnetic particles can be washed, for example, by the aforementioned washing methods.

Measurement of the label in step (2) can be carried out, for example, by the aforementioned methods.

The concentration of the component to be measured in a sample, based on the measured values obtained from measurements of the immunocomplexes formed on the magnetic particles, can be determined, for example, by methods similar to the aforementioned case of (I-2).

### (II-3) Competition method 2

Examples of the method for measuring a component to be measured in a sample by a competition method include methods that use a competitive substance that competes with the component to be measured in the antigen-antibody reaction as the protein in the protein-coupled magnetic particles of the present invention, and comprising the following steps of:
(1) reacting, in an aqueous medium, the component to be measured in a sample with magnetic particles to which the competitive substance is fixed, and a labeled antibody in which a label is bound to an antibody that binds to both the component to be measured and the competitive substance; and
(2) measuring the label in the immunocomplex of the labeled antibody and the competitive substance, which was formed in step (1).

After step (1), a step of washing the magnetic particles that have been applied for the antigen-antibody reaction can be set up. The magnetic particles can be washed, for example, by the aforementioned washing methods.

Measurement of the label in step (2) can be carried out, for example, by the aforementioned methods.

The concentration of the component to be measured in a sample, based on the measured values obtained from measurements of the immunocomplexes formed on the magnetic particles, can be determined by methods similar to the above-described case of (I-3).

Instead of the biotinylated protein, a biotinylated hydrocarbon compound can be used to measure the component to be measured in a sample. Examples of the component to be measured include a hydrocarbon compound constituting the biotinylated hydrocarbon compound, and an antibody that binds to the hydrocarbon compound. Examples of the hydrocarbon compound include the aforementioned hydrocarbon compounds. Measurement of the component to be measured in a sample using the biotinylated hydrocarbon compound can be performed using methods such as the aforementioned Sandwich method (I-1) or (II-1), and the competition method (I-3) or (II-3). In this case, a hydrocarbon compound is used instead of the first antibody of Sandwich methods (I-1) and (II-1), and a hydrocarbon compound is used as the competitive substance in competition method (I-3) or (II-3).

Furthermore, the component to be measured in a sample can be measured using a biotinylated nucleic acid instead of the biotinylated protein. Examples of the component to be measured include a nucleic acid that binds to a nucleic acid constituting the biotinylated nucleic acid and a protein that binds to a nucleic acid constituting the biotinylated nucleic acid. Examples of the protein include the aforementioned proteins. Measurements of the component to be measured in a sample using a biotinylated nucleic acid can be carried out by ordinary nucleic acid measurement methods, the aforementioned measurement methods, and such.

### 5. Reagents for measuring the component to be measured

An embodiment of the reagent for measuring the component to be measured of the present invention is a reagent comprising the streptavidin-coupled magnetic particles of the present invention and a biotinylated protein. Another embodiment of the reagent for measuring the component to be measured of the present invention is a reagent comprising the protein-coupled magnetic particles of the present invention. The reagent for measuring the component to be measured of the present invention is used in the method for measuring the component to be measured of the present invention. Examples of the protein in the biotinylated protein and the protein-coupled magnetic particles include the aforementioned proteins.

Furthermore, the component to be measured in a sample can be measured using a reagent comprising streptavidin-coupled magnetic particles of the present invention and a biotinylated hydrocarbon compound, or a reagent comprising hydrocarbon compound-coupled magnetic particles manufactured from streptavidin-coupled magnetic particles of the present invention and a biotinylated hydrocarbon compound. Examples of the component to be measured include a hydrocarbon compound constituting the biotinylated hydrocarbon compound and an antibody that binds to the hydrocarbon compound. Examples of the hydrocarbon compound include the aforementioned hydrocarbon compounds.

Furthermore, the component to be measured in a sample can be measured using a reagent comprising streptavidin-coupled magnetic particles of the present invention and a biotinylated nucleic acid, or a reagent containing nucleic acid-coupled magnetic particles manufactured from streptavidin-coupled magnetic particles of the present invention and a biotinylated nucleic acid. Examples of the component to be measured include a nucleic acid that binds to a nucleic acid constituting the biotinylated nucleic acid and a protein that binds to a nucleic acid constituting the biotinylated nucleic acid. Examples of the protein include the aforementioned proteins.

The measurement reagent of the present invention can include, as necessary, a component comprised in a reagent for immunological measurements used in ordinary immunoassays. Examples of the component include an aqueous medium, a salt, a metal ion, a sugar, an antiseptic agent, an agent for suppressing non-specific reaction, a surfactant, and an enzyme stabilizer. Examples of the aqueous medium include the aforementioned aqueous media. Examples of the salt include lithium chloride, sodium chloride, potassium chloride, calcium chloride, magnesium chloride, ammonium chloride, lithium bromide, sodium bromide, potassium bromide, calcium bromide, magnesium bromide, and ammonium bromide. Examples of the metal ion include magnesium ion, manganese ion, and zinc ion. Examples of the sugar include mannitol and sorbitol. Examples of the antiseptic agent include sodium azide, antibiotics (streptomycin, penicillin, gentamicin, etc.), BioAce, and Proclin 300. Examples of the agent for suppressing non-specific reaction include bovine serum albumin (BSA), fetal bovine serum (FBS), casein, and BlockAce (manufactured by Dainippon Pharmaceutical Co., Ltd.). Examples of the surfactant include a cationic surfactant, an anionic surfactant, an amphoteric surfactant, and a nonionic surfactant. Examples of the enzyme stabilizer include peroxidase stabilizing buffer and alkaline phosphatase stabilizing buffer.

Herein below, the present invention will be specifically described with reference to the Examples, which is not to be construed as limiting the scope of the present invention.

### [Example 1]

### (1) Manufacturing of streptavidin-coupled magnetic particles

Amino group-type Estapor magnetic particles EM2-100/40 (manufactured by Merck) were used for the magnetic particles. The magnetic particles are composed of a core shell structure and have a particle size of 1.62 µm, and they are particles in which the inner core contains a magnetic material which accounts for 41.2% of the total weight, and in which the shell portion comprising polystyrene is chemically modified to have 97 µeq/g of amino groups. 2 mg of the magnetic particles were collected, and 4 mL of a 10 mmol/L acetate buffer at pH5.5 containing 1.0% trimethylstearylammonium chloride (manufactured by Tokyo Chemical Industry Co., Ltd.) (hereinafter referred to as dispersion liquid A) was added for dispersion. Subsequently, the magnetic particles were collected by positioning a powerful magnet to the side of the container, and dispersion liquid A was removed by aspiration (hereinafter, the sequence of operations consisting of addition of dispersion liquid, collection of magnetic particles, and removal by aspiration will be abbreviated as "washing"). After washing for four successive times, the particles were suspended in a 10 mmol/L acetate buffer at pH5.5 to prepare a 30 mg/mL suspension solution of magnetic particles. Solutions of glutaraldehyde at 0.038%, 0.063%, 0.088%, and 0.113% were prepared by diluting a 25% aqueous glutaraldehyde solution (manufactured by Nacalai Tesque) with a pH5.5 10 mmol/L acetate buffer. Recombinant streptavidins (manufactured by Roche) were used for streptavidin, and a streptavidin solution was prepared by dissolving them in a pH5.5 10 mmol/L acetate buffer at 24.5 mg/mL. The solution was left to stand at ice-cold temperature for one hour or more.

Next, 30 µL of the streptavidin solution was added to 66.7 µL of the particle-dispersed liquid, then 30 µL of the 0.038% glutaraldehyde solution was added, and this was stirred using a wave rotor at 35°C for 20 hours. Similar operations were carried out for the glutaraldehyde solutions having concentrations of 0.063%, 0.088%, and 0.113%.

The obtained particles were washed ten times using a 50 mmol/L MES buffer (pH6.5) containing 1.0% BSA and 0.09% sodium azide to obtain streptavidin-coupled magnetic particles.

### (2) Measurement of biotin-binding capacity of streptavidin-coupled magnetic particles

The biotin-binding capacities were determined for the above-mentioned streptavidin-coupled magnetic particles obtained in (1) and for commercially available streptavidin-coupled magnetic particles by the following method.

Streptavidin-coupled magnetic particles were dispersed at 1 mg/mL in 0.1 % BSA-PBS [PBS: a 10 mmol/L phosphate buffer (pH7.2) containing 0.15 mol/L sodium chloride], and the dispersion was diluted by a serial two-fold dilution method up to six steps (64-fold dilution) to reach 0.0156 mg/mL. The six samples and a blank (0.1 % BSA-PBS) were each dispensed into a 96-well black plate in 50-µL aliquots. Next, Biotin-Fluorescein (manufactured by Thermo Scientific) was diluted to 1 µg/mL using 0.1 % BSA-PBS, and this was dispensed in 50-µL aliquots to the wells containing the dispensed samples. The plate containing the dispensed samples was incubated at 37°C for ten minutes while shaking using a shaker-incubator (manufactured by Amalyte), and the fluorescence intensities of the dispersed particles were measured with a fluorescence plate reader "Plate Chameleon V" (manufactured by HIDEX).

When fluorescence-labeled biotins bind to streptavidins on the magnetic particles at this point, the fluorescence-labeled biotins bound to streptavidins will exist close to each other, and fluorescence quenching occurs. Fluorescence quenching increases as the amount of fluorescence-labeled biotin bound to the streptavidin coupled to the magnetic particles increases per unit area. Using this property, the fluorescence reduction rate of streptavidin was evaluated in advance using commercially available magnetic particles with known biotin-binding capacity as the reference, and the biotin-binding capacity of the streptavidin-coupled magnetic particles of the present invention was calculated. In this Example, the concentration of streptavidin-coupled magnetic particles when the fluorescence intensity decreased by 50% was calculated by straight-line approximation from the dilution samples of streptavidin-coupled magnetic particles, and the biotin-binding capacities (pmol/mm²) were calculated by comparison to the reference streptavidin.

The measurement results are shown in Table 1.

**Table 1**

| Streptavidin-coupled magnetic particles | Preparation condition (glutaraldehyde solution concentration) | Biotin-binding capacity (pmol/mm²) |
|---|---|---|
| Example 1 | 0.038% | 2.28 |
| | 0.063% | 4.83 |
| | 0.088% | 6.17 |
| | 0.113% | 7.15 |
| Commercially available product | Dynabeads T1 manufactured by Dynal | 0.36 |
| | BE-M08/10 manufactured by Merck | 0.39 |

As it is clear from Table 1, the above-mentioned streptavidin-coupled magnetic particles obtained in (1) were found to have high biotin-binding capacity compared to commercially available streptavidin-coupled magnetic particles (Dynabeads T1 manufactured by Dynal and BE-M08/10 manufactured by Merck).

### [Example 2]

### Analysis of cross-linked structure of streptavidin on the magnetic particles

Streptavidin-coupled magnetic particles having biotin-binding capacities of 2.61 pmol/mm², 4.95 pmol/mm², and 6.76 pmol/mm², which were obtained by a method similar to the method of Example 1, were used. The respective streptavidin-coupled magnetic particles were washed ten times using 4 mL of PBS, and after substitution of PBS with 1% SDS/PBS, incubation was carried out at 60°C for one hour. Next, the magnetic particles were collected using a magnet, and after collecting the supernatant protein solution, the supernatant was subjected to analysis by SDS-PAGE. A similar operation was carried out on commercially available streptavidin-coupled magnetic particles. The SDS-PAGE result is shown in Fig. 1.

As it is clear from Fig. 1, while only monomers constituting streptavidin were observed in streptavidin and commercially available streptavidin-coupled magnetic particles, bands of dimers, trimers, tetramers, and higher-order multimers were observed in addition to the monomer band in the streptavidin-coupled magnetic particles of the present invention. This revealed that in commercially available streptavidin-coupled magnetic particles, the streptavidins do not form a cross-linked structure, whereas in the streptavidin-coupled magnetic particles of the present invention, the streptavidins form a cross-linked structure.

### [Test Example 1] Evaluation of dispersibility of streptavidin-coupled magnetic particles

Streptavidin-coupled magnetic particles having a biotin-binding capacity of 4.54 pmol/mm², which were prepared by a method similar to Example 1, were dispersed at 0.1 mg/mL in PBS containing 0.1% BSA, and this was left to stand in a cool dark place for 24 hours. After stirring by inversion for 25 times, the degrees of dispersion were compared. The results are shown in Fig. 2. In Fig. 2, the top photograph shows the static state of the streptavidin-coupled magnetic particles, and the bottom photograph shows the dispersed state of the streptavidin-coupled magnetic particles after mixing by inversion for 25 times.

As it is clear from Fig. 2, streptavidin-coupled magnetic particles of the present invention were found to have very good dispersion properties.

### Industrial Applicability

The present invention provides a streptavidin-coupled magnetic particle having high biotin-binding capacity and a manufacturing method thereof, a protein-coupled magnetic particle manufactured using the streptavidin-coupled magnetic particle and a manufacturing method thereof, a method for measuring a component to be measured, and a reagent for measuring a component to be measured. The streptavidin-coupled magnetic particle and protein-coupled magnetic particle manufactured by the manufacturing method of the present invention, as well as the method for measuring a component to be measured and the reagent for measuring a component to be measured of the present invention are useful in clinical diagnosis.

## Claims

1. A streptavidin-coupled magnetic particle, having a structure in which streptavidins are cross-linked with each other on a magnetic particle.

2. The streptavidin-coupled magnetic particle of claim 1, which is manufactured by a method comprising the following steps of:
(1) preparing a suspension comprising magnetic particles having amino groups on their surface; and
(2) reacting the magnetic particles with streptavidin and glutaraldehyde by adding glutaraldehyde in the presence of streptavidin to the suspension prepared in step (1).

3. The streptavidin-coupled magnetic particle of claim 2, which is manufactured by a method further comprising the following step (3):
(3) reacting the streptavidin-coupled magnetic particles prepared in step (2) with a reducing agent.

4. A protein-coupled magnetic particle, which is manufactured using the streptavidin-coupled magnetic particle of any one of claims 1 to 3 and a biotinylated protein.

5. A method for measuring a component to be measured in a sample, which uses the protein-coupled magnetic particle of claim 4.

6. A method for measuring a component to be measured in a sample, which uses the streptavidin-coupled magnetic particle of any one of claims 1 to 3 and a biotinylated protein.

7. A reagent for measuring a component to be measured in a sample, which comprises the protein-coupled magnetic particle of claim 4.

8. A reagent for measuring a component to be measured in a sample, which comprises the streptavidin-coupled magnetic particle of any one of claims 1 to 3 and a biotinylated protein.

9. A method for manufacturing streptavidin-coupled magnetic particles, which comprises the following steps of:
(1) preparing a suspension comprising magnetic particles having amino groups on their surface; and
(2) reacting the magnetic particles with streptavidin and glutaraldehyde by adding glutaraldehyde in the presence of streptavidin to the suspension prepared in step (1).

10. The manufacturing method of claim 9, further comprising the following step (3):
(3) reacting the streptavidin-coupled magnetic particles prepared in step (2) with a reducing agent.

11. A method for manufacturing protein-coupled magnetic particles, which comprises the following steps of:
(1) preparing a suspension comprising magnetic particles having amino groups on their surface;
(2) preparing streptavidin-coupled magnetic particles by adding glutaraldehyde in the presence of streptavidin to the suspension prepared in step (1); and
(3) reacting the streptavidin-coupled particles prepared in step (2) with a biotinylated protein.

12. A method for manufacturing protein-coupled magnetic particles, which comprises the following steps of:
(1) preparing a suspension comprising magnetic particles having amino groups on their surface;
(2) preparing streptavidin-coupled magnetic particles by adding glutaraldehyde in the presence of streptavidin to the suspension prepared in step (1);
(3) reacting the streptavidin-coupled magnetic particles prepared in step (2) with a reducing agent; and
(4) reacting the streptavidin-coupled magnetic particles prepared in step (3) with a biotinylated protein.
